# EUROPEAN PATENT SPECIFICATION

(11) **EP 2 252 279 B1**
(45) Date of publication and mention of the grant of the patent: **11.09.2013**
(21) Application number: 08802695.0
(22) Date of filing: 29.09.2008
(51) Int. Cl.: A61K 31/167, A61K 31/195, A61K 31/198, A61P 39/02, A61P 1/16

(54) **AMINO ACID FORMULATIONS COMPRISING CYSTEINE, METHIONINE AND SERINE FOR THE PREVENTION OF PARACETAMOL-INDUCED LIVER DAMAGE**
AMINOSÄURE-FORMULIERUNGEN MIT CYSTEIN, METHIONIN UND SERIN ZUR PRÄVENTION VON PARACETAMOL-INDUZIERTER LEBERSCHÄDIGUNG
FORMULATIONS D'ACIDES AMINÉS COMPRENANT LA CYSTÉINE, LA MÉTHIONINE ET LA SÉRINE POUR LA PRÉVENTION D'UNE ATTEINTE HÉPATIQUE INDUITE PAR LE PARACÉTAMOL

(30) Priority: 07.02.2008 IT MI20080187
(43) Date of publication of application: 24.11.2010
(73) Proprietor: Velleja Research SRL, 29010 Pontenure (PC) (IT)
(72) Inventor: DI PIERRO, Francesco, I-29010 Pontenure (PC) (IT)
(74) Representative: Minoja, Fabrizio
(86) International application number: PCT/EP2008/008261
(87) International publication number: WO 2009/097874

(56) References cited:
- WO-A-01/68069
- WO-A-94/08628
- WO-A-94/20086
- WO-A-98/32434
- WO-A-2005/097120
- WO-A-2008/073344
- FR-A- 2 744 917
- US-A1- 2007 099 843
- MCLEAN A M ET AL: "The effect of diet on the toxicity of paracetamol and the safety of paracetamol-methionine mixtures" BIOCHEMICAL PHARMACOLOGY, PERGAMON, OXFORD, GB, vol. 24, no. 1, 1 January 1975 (1975-01-01), pages 37-42, XP023847723 ISSN: 0006-2952 [retrieved on 1975-01-01]
- TERNEUS ET AL: "Comparison of S-adenosyl-l-methionine (SAMe) and N-acetylcysteine (NAC) protective effects on hepatic damage when administered after acetaminophen overdose" TOXICOLOGY, LIMERICK, IR, vol. 244, no. 1, 7 November 2007 (2007-11-07), pages 25-34, XP022401878 ISSN: 0300-483X

## Description

Compositions comprising a combination of amino acids for the prevention of paracetamol-induced liver damage.

### Background of the invention

Paracetamol, or acetaminophen, was one of the first non-steroidal antiinflammatory drugs (NSAIDs) to be used, especially as an antipyretic and analgesic. Unlike other substances in the same family, paracetamol is well tolerated at gastric level. Its main side effect is the risk of liver toxicity caused by centrilobular necrosis, especially following an accidental overdose. Moreover, many drugs containing paracetamol, especially those for paediatric use, are presented in forms such as syrup or drops, which do not guarantee a sufficiently precise dose. Another possible risk derives from chronic use of the product (e.g. by elderly people who suffer from chronic bone and/or muscle pain which fails to respond to NSAIDs, due to the risk of gastric lesions).

The problem is a major one in view of the fact that in an urban area with 2 million inhabitants, up to 5 cases a day are described in the paediatric field alone. 70-100,000 cases a year of paracetamol-induced liver disease are reported in the UK alone. Finally, doses exceeding four grams a day (for an adult weighing 80 kg or more) are considered highly dangerous, with potentially fatal liver toxicity.

Paracetamol is metabolised by the cells of zone 3 of the liver acinus by cytochrome P450, which transforms the paracetamol into L-Cysteine or N-acetylcysteine as precursor of L-Cysteine, with L-Methionine or S-adenosylmethionine as transporter of L-Methionine and L-Serine or phosphoserine as precursor of L-Serine N-acetylbenzoquinoneimine, a highly reactive, toxic compound, which mainly affects the liver proteins. The cells of zone 3 of the liver acinus conjugate the toxic metabolite with glutathione (GSH) to promote its elimination. When the GSH reserves have been used up, the toxic compound accumulates in the hepatocyte, causing its death. The cells of zone 1 of the liver acinus could also conjugate paracetamol with GSH to form a GSH-conjugate which is readily eliminated, but less efficiently, due to the lower concentration of glutathione in said cells.

Paracetamol, as well as being a substrate of cytochrome P450, can also be transformed to N-acetylbenzosemiquinimine, a radical toxic to the cells of the renal medulla, causing lipid peroxidation and renal necrosis.

Compositions comprising L-methionine, N-acetylcysteine or L-cysteine or mixtures thereof for the prevention of paracetamol induced liver damages are known (WO94/08628). L-methionine, N-acetylcysteine and L-cysteine can be used alone for the prevention of paracetamol-induced liver damages, either in separate dosage units or formulated together with paracetamol (WO94/20086, WO98/32434, Mac Lean, A.E.M. et al., Biochem. Pharmacol. 1975, 24, 37-42, WO01/68069, US2007/099843, W02005/097120, FR 2 744 917).

In case of poisoning, high doses of N-acetylcysteine (NAC) i.v. are used as antidote (140 mg/kg as loading dose, followed by 70 mg/kg every 4 hours). If the maximum dose is reached, in the absence of liver disease, administration of the same substance is recommended, at standard doses and procedures. NAC is effective if it is administered within 8 hours after the last administration of paracetamol; after 24 hours, NAC is ineffective as an antidote.

N-acetylcysteine possesses an antioxidant action: for example, it protects alpha-antitrypsin, an elastase-inhibiting enzyme, from inactivation by hypochlorous acid (HOCI), a very powerful oxidising agent produced by the enzyme myeloperoxidase secreted by activated phagocytes.

In the cell, NAC is deacetylated, releasing L-Cysteine, an amino acid essential for the synthesis of cell GSH. GSH is a tripeptide whose synthesis is limited by the availability of L-Cysteine.

NAC performs a crucial role in the maintenance of adequate GSH levels, helping to protect the cell against various harmful toxic agents. It has been demonstrated that paracetamol-induced damage is caused by a GSH deficiency in the hepatocyte. The toxic metabolite of paracetamol is removed by conjugation with GSH, thus preventing centrilobular damage and damage to the renal parenchyma.

L-Methionine, a sulphated amino acid, also plays a part in the biosynthesis of GSH, though indirectly, through the synthesis of L-Cysteine. It has been reported that L-Methionine, if used at the same dose as L-Cysteine, antagonises centrilobular liver necrosis and the kidney damage caused by paracetamol due to its ability to induce neo-synthesis of L-Cysteine (Terneus M.V. et al., J Pharmacol Exp Ther 2007; 320: 99-107), which in turn contributes to the synthesis of GSH. However, by contributing directly to the synthesis of GSH, L-Cysteine induces more rapid replenishment of GSH than the administration of L-Methionine.

In order to replace the depleted GSH it is therefore possible to administer NAC, an efficient transporter of L-Cysteine as is, and the "L-Cysteine-precursor" L-Methionine. Administration of L-Methionine alone, however, may not be sufficient to restore the whole of the necessary pool of L-Cysteine, a limiting factor in the biosynthesis of GSH. L-Methionine is only able to provide the -SH group for the synthesis of L-Cysteine, not the carbon skeleton. The latter is supplied by the amino acid L-Serine.

### Description of the invention

It has now been found that combinations of: are surprisingly more effective, useful dose being equal, in counteracting the toxic effects of paracetamol than the administration of NAC alone (or another cysteine transporter, such as L-Cystine, or L-Cysteine as L-Cysteine or N-acetylcysteine as precursor of L-Cysteine, with L-Methionine or S-adenosylmethionine as transporter of L-Methionine and L-Serine or phosphoserine as precursor of L-Serine is), or L-Methionine alone.

The administration of the three L-amino acids (or their transporters/precursors or mixtures thereof) is preferred, the L-Cysteine providing at the first stage an immediately available precursor of liver GSH, while the L-Methionine/L-Serine pair causes protracted biosynthesis of other L-Cysteine still usable by the cell for GSH synthesis.

The invention therefore provides paracetamol compositions comprising:

N-acetylcysteine can be conveniently used as a source of L-Cysteine (as can L-Cystine); S-adenosylmethionine or SAMe is an example of a precursor or transporter of L-Methionine, while phosphoserine is an example of a precursor or transporter of L-Serine.

The association according to the invention totally counteracts the damage to the hepatic and renal parenchyma caused by paracetamol.

The association is effective if co-administered with paracetamol for dose intervals ranging between 5 and 200% (preferably 20%) of the dose of paracetamol administered. Every 1000 mg of paracetamol, 50 to 2000 mg (preferably 200 mg) of pure amino acids should therefore be administered.

L-Cysteine or N-acetylcysteine as precursor of L-Cysteine, with L-Methionine or S-adenosylmethionine as transporter of L-Methionine and L-Serine or phosphoserine as precursor of L-Serine.

In particular, the formulations according to the invention may contain (per treatment/day):
L-Cysteine: from 0.1 to 8000 mg
L-Methionine: from 0.1 to 8000 mg
L-Serine: from 0.1 to 8000 mg
NAC: from 0.1 to 10000 mg
SAMe: from 0.1 to 10000 mg
Phosphoserine: from 0.1 to 5000 mg.

The association of the three amino acids is effective if administered separately, in a different dose form, or in a single dose form including paracetamol.

### Examples of formulations (mg/dosage unit):

### Example no. 1:

| | |
|---|---|
| Paracetamol | 500 mg |
| L-Cysteine | 100 mg |
| L-Methionine | 100 mg |
| L-Serine | 100 mg |

### Example no. 2:

| | |
|---|---|
| Paracetamol | 500 mg |
| NAC | 300 mg |
| L-Methionine | 100 mg |
| L-Serine | 100 mg |

### Example no. 3:

| | |
|---|---|
| Paracetamol | 500 mg |
| NAC | 200 mg |
| SAMe | 250 mg |
| L-Serine | 100 mg |

### Example no. 4: Reference example

| | |
|---|---|
| Paracetamol | 500 mg |
| NAC | 400 mg |
| SAMe | 250 mg |

### Example no. 5:

| | |
|---|---|
| Paracetamol | 500 mg |
| NAC | 400 mg |
| SAMe | 125 mg |
| Phosphoserine | 125 mg |

### Example no. 6: C Reference example

| | |
|---|---|
| Paracetamol | 500 mg |
| NAC | 400 mg |
| L-Methionine | 125 mg |

### Example no. 7: Reference example

| | |
|---|---|
| Paracetamol | 500 mg |
| NAC | 400 mg |
| L-Serine | 250 mg |

### Example no. 8: Reference example

| | |
|---|---|
| Paracetamol | 500 mg |
| NAC | 400 mg |
| Phosphoserine | 250 mg |

## Claims

1. Compositions comprising a combination of amino acids:
L-Cysteine or N-acetylcysteine as precursor of L-Cysteine, with L-Methionine or S-adenosylmethionine as transporter of L-Methionine and L-Serine or phosphoserine as precursor of L-Serine;
for use in the prevention of paracetamol-induced liver damage.

2. Compositions for use according to claim 1, wherein paracetamol is present in the same dosage unit as the amino acids.

3. Compositions for use according to claim 1, including 50 to 2000 mg of pure amino acids per 1000 mg of paracetamol.

## Patentansprüche

1. Zusammensetzungen umfassend eine Kombination von Aminosäuren:
L-Zystein oder N-Acetylzystein als Vorstufe von L-Zystein, mit L-Methionin oder S-Adenosylmethionin als Transporter von L-Methionin und L-Serin oder Phosphoserin als Vorstufe von L-Serin; zur Verwendung in der Prävention von Paracetamolinduzierter Leberschädigung.

2. Zusammensetzungen zur Verwendung gemäß Anspruch 1, wobei Paracetamol in der gleichen Dosierungseinheit wie die Aminosäuren vorhanden ist.

3. Zusammensetzungen zur Verwendung gemäß Anspruch 1, beinhaltend 50 bis 2000 mg pure Aminosäuren pro 1000 mg Paracetamol.

## Revendications

1. Compositions comprenant une combinaison d'acides aminés :
de la L-cystéine ou de la N-acétylcystéine comme précurseur de L-cystéine, avec de la L-méthionine ou de la S-adénosylméthionine comme transporteur de L-méthionine et de la L-sérine ou de la phosphosérine comme précurseur de L-sérine ;
pour une utilisation dans la prévention des lésions du foie induites par le paracétamol.

2. Compositions pour l'utilisation selon la revendication 1, dans lesquelles le paracétamol est présent dans la même unité posologique que les acides aminés.

3. Compositions pour l'utilisation selon la revendication 1, incluant 50 à 2 000 mg d'acides aminés purs pour 1 000 mg de paracétamol.
